# EUROPÄISCHE PATENTSCHRIFT

(11) **EP 4 031 108 B1**
(45) Veröffentlichungstag und Bekanntmachung des Hinweises auf die Patenterteilung: **02.04.2025**
(21) Anmeldenummer: 20780970.8
(22) Anmeldetag: 18.09.2020
(51) Int. Cl.: A61K 9/00, A61K 9/16, A61K 9/14, A61K 38/00

(54) **EXTRUDIERTE DEPOTFORM MIT KONTROLLIERTER WIRKSTOFFFREISETZUNG**
EXTRUDED DEPOT FORM FOR CONTROLLED ACTIVE SUBSTANCE RELEASE
FORME DE DÉPÔT EXTRUDÉE POUR LA LIBÉRATION CONTRÔLÉE DE SUBSTANCE ACTIVE

(30) Priorität: 19.09.2019 DE 102019125208
(43) Veröffentlichungstag der Anmeldung: 27.07.2022
(73) Patentinhaber: AMW GmbH, 83627 Warngau (DE)
(72) Erfinder: RITTER, Elisabeth, 83627 Warngau (DE); FEUERSINGER, Alexandra, 83059 Kolbermoor (DE); OLIV, Lukas, 83700 Rottach-Egern (DE); RADDATZ, Klaus, 89551 Königsbronn (DE); BARTH, Dirk, 82166 Gräfelfing (DE)
(74) Vertreter: Beckord & Niedlich Patentanwälte PartG mbB
(86) Internationale Anmeldenummer: PCT/EP2020/076148
(87) Internationale Veröffentlichungsnummer: WO 2021/053167

(56) Entgegenhaltungen:
- EP-A1- 0 486 959
- WO-A1-00/03660
- WO-A1-03/075892
- WO-A1-2018/172494
- US-A1- 2008 145 403
- SENGUPTA SHILADITYA ET AL: "Temporal targeting of tumour cells and neovasculature with a nanoscale delivery system", NATURE, MACMILLAN JOURNALS LTD, LONDON, vol. 436, no. 7050, 28 July 2005 (2005-07-28), pages 568 - 572, XP037238298, ISSN: 0028-0836, DOI: 10.1038/NATURE03794
- QI WANG ET AL: "Core shell lipid-polymer hybrid nanoparticles with combined docetaxel and molecular targeted therapy for the treatment of metastatic prostate cancer", SCIENTIFIC REPORTS, vol. 7, no. 1, 19 July 2017 (2017-07-19), XP055691663, DOI: 10.1038/s41598-017-06142-x
- RONNIE H. FANG ET AL: "Lipid-insertion enables targeting functionalization of erythrocyte membrane-cloaked nanoparticles", NANOSCALE, vol. 5, no. 19, 17 July 2013 (2013-07-17), United Kingdom, pages 8884 - 8888, XP055573231, ISSN: 2040-3364, DOI: 10.1039/c3nr03064d

## Beschreibung

Die vorliegende Erfindung betrifft eine extrudierte Depotform, umfassend zumindest einen Wirkstoff und zumindest eine erste Verbindung aus der Klasse der bioabbaubaren organischen Polymere und zumindest eine zweite Verbindung aus der Klasse der Lipide. Des Weiteren betrifft die vorliegende Erfindung ein Verfahren zur Herstellung der extrudierten Depotform sowie die Verwendung der extrudierten Depotform.

Subkutane Darreichungsformen umfassen im Allgemeinen flüssige oder feste Formulierungen, die per Injektion oder chirurgischen Eingriff in das subkutane Gewebe verabreicht werden können. Feste Formulierungen werden dabei zumeist als zylinderförmige Polymerstäbchen mit darin eingebettetem Wirkstoff verabreicht. Darreichungsformen, die dabei Wirkstoffe über einen Zeitraum von mehreren Tagen bis zu beispielsweise 24 Monaten abgeben, werden auch als Depotarzneiformen bezeichnet.

Von Depotarzneiformen abgegebene Wirkstoffe unterliegen nicht dem sogenannten First-Pass-Effekt, passieren also nicht den Verdauungstrakt und die Leber und können zudem eine kontinuierliche Wirkstoffabgabe über einen anhaltenden Zeitraum gewährleisten. Auf diese Weise werden starke Schwankungen der Wirkstoffkonzentration und damit einhergehende Nebenwirkungen vermieden, welche beispielsweise häufig bei intravenösen Darreichungsformen auftreten. Die kontrollierte, dauerhafte Wirkstofffreisetzung von Depotarzneiformen (im Folgenden auch "Depotformen" genannt) kann zudem das Applikationsintervall verlängern. Ferner müssen Depotarzneiformen, die bioabbaubar sind, nach der beabsichtigen Applikationszeit nicht mehr entfernt werden. Diese Eigenschaften machen subkutane Depotformen zu anwenderfreundlichen Arzneimitteln.

WO2018/172494 enthüllt Depotformen, die bio-abbaubare organische Polymere und Lipide umfassen können.

EP0486959 enthüllt extrudierte Depotformen, die als Mikropartikel bereitgestellt werden.

US2008/145403 beschreibt Depotformen, die bio-abbaubare organische Polymere und zyklische Lipide umfassen können.

Die Wirkstofffreisetzungsrate und -dauer aus Depotformen kann durch in der Formulierung enthaltene Zusatzstoffe beeinflusst werden, wobei für Formulierungen mit langer Applikationsdauer hohe Anforderungen an die Biokompatibilität der Inhaltsstoffe bestehen, um gesundheitliche Beeinträchtigungen von Patienten zu minimieren.

Daher werden Formulierungen benötigt, welche eine kontinuierliche, kontrollierte Abgabe über einen langen Applikationszeitraum und eine hohe Biokompatibilität sowie gute Eigenschaften im Hinblick auf deren Bioabbaubarkeit aufweisen.

Die im Stand der Technik beschriebenen Darreichungssysteme weisen kurze Applikationszeiten auf und/oder verfehlen gar die erforderliche therapeutische Wirkstoffdosis beim Anwender.

Es ist daher die Aufgabe der vorliegenden Erfindung, eine bioabbaubare Depotform für die parenterale Verabreichung von Wirkstoffen vorzusehen, welche eine anhaltende, kontrollierte Wirkstofffreisetzung in einer für die Therapie geeigneten Dosis ermöglicht.

Diese Aufgabe wird erfindungsgemäß durch eine extrudierte Depotform gelöst, welche zumindest einen Wirkstoff und zumindest eine Verbindung aus der Klasse der bioabbaubaren organischen Polymere und zumindest eine zweite Verbindung aus der Klasse der Lipide gemäß Anspruch 1 umfasst, sowie durch ein Verfahren zur Herstellung der erfindungsgemäßen Depotform gemäß Anspruch 10. Des Weiteren wird die Aufgabe durch eine erfindungsgemäße Zusammensetzung zur Verwendung gemäß Anspruch 13 13 und/oder 14 gelöst.

Die vorliegende Erfindung betrifft daher eine extrudierte Depotform zur anhaltenden Wirkstofffreisetzung umfassend
(a) zumindest einen Wirkstoff,
(b) zumindest eine erste Verbindung aus der Klasse der bioabbaubaren organischen Polymere auf Basis von Milchsäure und/oder Glykolsäure, und
(c) zumindest eine zweite Verbindung aus der Klasse der Lipide, und
(d) wobei b) und c) vorzugsweise mindestens 50 Gew.-% des Trockengewichts der Depotform umfassen, wobei das Verhältnis der zumindest einen Verbindung aus der Klasse der bioabbaubaren organischen Polymere und der zweiten Verbindung aus der Klasse der Lipide bei höchstens etwa 25:1 und/oder bei mindestens etwa 5:1 liegt,wobei der Schmelzpunkt der zumindest einen Verbindung aus der Klasse der Lipide bei mindestens etwa 60 °C und/oder bei höchstens etwa 80 °C liegt,und wobei die Depotform eine Länge von mindestens 0,1 cm und/oder von höchstens 5 cm aufweist.

Unter der Bezeichnung "Depotform" ist vorliegend eine Arzneiform zu verstehen, bei welcher die Wirkstofffreisetzung durch Retardierung über einen längeren Zeitraum erfolgt. Erfindungsgemäß erfolgt die Verabreichung der Depotform parenteral und bildet ein vorzugsweise subkutanes Depot.

Unter der Bezeichnung "bioabbaubar" ist zu verstehen, dass in der Formulierung enthaltene Substanzen *in vivo* beispielsweise durch enzymatische, chemische oder physikalische Prozesse in kleinere Einheiten abgebaut werden bzw. erodieren.

In ihrer einfachsten Ausführungsform umfassen die erfindungsgemäßen Depotformen zumindest einen Wirkstoff und zumindest eine erste Verbindung aus der Klasse der bioabbaubaren organischen Polymere auf Basis von Milchsäure und/oder Glykolsäure und zumindest eine zweite Verbindung aus der Klasse der Lipide.

Die kontrollierte Abgabe des zumindest einen Wirkstoffs aus der extrudierten Depotform kann erfindungsgemäß durch die Kombination aus einem bioabbaubaren organischen Polymer auf Basis von Milchsäure und/oder Glykolsäure und einem Lipid signifikant verbessert werden. So weisen erfindungsgemäße extrudierte Depotformen vorteilhaft eine kontrollierte Wirkstoffabgabe in einem Zeitraum von einer Woche bis zu einem Jahr, eine hohe Biokompatibilität und eine gute Bioabbaubarkeit auf.

Dabei weist das Trockengewicht der zumindest einen Verbindung aus der Klasse der bioabbaubaren organischen Polymere auf Basis von Milchsäure und/oder Glykolsäure und der zumindest einen Verbindung aus der Klasse der Lipide einen Anteil von mehr als etwa 50 Gew.-%, bevorzugt von mehr als etwa 55 Gew.-%, besonders bevorzugt von mehr als etwa 60 Gew.-%, insbesondere bevorzugt von mindestens etwa 62 Gew.-%, am Gesamtgewicht der Depotform auf.

Das Trockengewicht der zumindest einen Verbindung aus der Klasse der bioabbaubaren Polymere und der zumindest einen Verbindung aus der Klasse der Lipide weist jedoch maximal einen Anteil von etwa 99 Gew.-%, bevorzugt von maximal etwa 97,5 Gew.-%, besonders bevorzugt von maximal etwa 95 Gew.-%, insbesondere bevorzugt von etwa 90 Gew.-%, am Gesamtgewicht der Depotform auf.

Die Bezeichnung "etwa" bedeutet, dass ein spezifizierter Messwert, wie zum Beispiel ein Gehalt in der Zusammensetzung, eine Abweichung des angegebenen Messwerts innerhalb der Messtoleranz einer geeigneten Messmethode haben kann.

Als Trockengewicht der Depotform wird vorliegend das Gewicht einer zur Darreichung bereiten Formulierung bezeichnet, die keinen bzw. einen vernachlässigbaren Gehalt, insbesondere weniger als etwa 3 Gew.-%, an Wasser aufweist.

Der Gesamtgehalt der zumindest einen Verbindung aus der Klasse der bioabbaubaren Polymere umfasst dabei den Gehalt aller Verbindungen aus der Klasse der bioabbaubaren Polymere und aus der Klasse der Lipide in der wirkstoffhaltigen Depotform.

In einer erfindungsgemäßen Depotform weist die zumindest eine zweite Verbindung aus der Klasse der Lipide einen Schmelzpunkt auf, welcher über Raumtemperatur, also einer Temperatur von etwa 25 °C, liegt. Vorzugsweise liegt der Schmelzpunkt des zumindest einen Lipids bei über etwa 40 °C, besonders bevorzugt bei über etwa 50 °C, insbesondere bei über etwa 60 °C, insbesondere bevorzugt bei über etwa 70 °C. Jedoch liegt der Schmelzpunkt des Lipids in der erfindungsgemäßen Depotform nicht über 100 °C.

Die erfindungsgemäße extrudierte Depotform gibt bei deren subkutaner Anwendung den zumindest einen Wirkstoff aus der wirkstoffhaltigen Depotform an das umgebende Gewebe ab, wobei vorzugsweise ein wesentlicher Teil des Wirkstoffs systemisch aufgenommen wird. Insofern die extrudierte Depotform für eine lokale Therapie vorgesehen ist, kann ein wesentlicher Anteil des Wirkstoffs vorteilhaft in das den Applikationsort umgebende Gewebe abgegeben werden.

Die in der Depotform enthaltene absolute Wirkstoffmenge bestimmt im Allgemeinen die Zeitspanne, in welcher eine kontinuierliche Zufuhr des Wirkstoffs in oder an den Organismus aufrechterhalten wird. Deshalb ist eine möglichst hohe Beladung der Depotform mit zumindest einem Wirkstoff dann wünschenswert, wenn die Applikationszeit der Depotform lang ist, d. h. mehrere Wochen bis zu zwölf Monaten beträgt.

Eine erfindungsgemäße extrudierte Depotform wird vorzugsweise für eine Applikationsdauer von mindestens einer Woche bis maximal 12 Monaten, bevorzugt für eine Applikationsdauer von einer Woche bis 6 Monaten, insbesondere für eine Applikationsdauer von 2 Wochen bis 3 Monaten, angewendet.

Die vorliegende Erfindung betrifft somit die medizinische, tiermedizinische und/oder kosmetische Verwendung der erfindungsgemäßen Depotform zur Abgabe von Wirkstoffen in den Blutkreislauf eines menschlichen oder tierischen Körpers.

Des Weiteren betrifft die vorliegende Erfindung ein Verfahren zur Herstellung einer erfindungsgemäßen Depotform, wobei das Verfahren folgende Schritte umfasst:
(i) Bereitstellen einer vorzugsweise homogenen Mischung umfassend zumindest einen Wirkstoff, zumindest eine erste Verbindungen aus der Klasse der bioabbaubaren organischen Polymere auf Basis von Milchsäure und/oder Glykolsäure und zumindest eine zweite Verbindung aus der Klasse der Lipide gemäss einem der Ansprüche 1-9,
(ii) Extrusion, insbesondere Schmelzextrusion, des Kerns bei einer Temperatur oberhalb der Schmelztemperatur des zumindest einen Lipids,
(iii) optional Aufbringen einer Überzugsmischung bzw. -zusammensetzung auf das vorstehend erhaltene Extrudat vorzugsweise gleichzeitig mit Schritt (ii),
(iv) Schneiden des Extrudats in Stücke geeigneter Größe,
(v) optional Runden der Stücke, insbesondere durch Sphäronisation,
(vi) optional Durchführen eines Sterilisierungsvorgangs und/oder Verpacken der Depotform.

Unter Bereitstellen wird dabei sowohl eine Herstellung vor Ort als auch ein Zuliefern einer homogenen Mischung verstanden. Hierbei kann eine homogene Mischung durch einen geeigneten Mischvorgang, bevorzugt ohne Zugabe von Lösungsmitteln, hergestellt werden. Der Mischvorgang kann zudem mehr als einen Schritt umfassen, indem beispielsweise zuerst eine Mischung aus dem bioabbaubaren Polymer und dem Lipid und, davon getrennt, eine Mischung eines oder mehrerer optionalen/r Hilfsstoffe/s und des zumindest einen Wirkstoffs angesetzt wird, welche in einem zweiten Schritt mit dem Polymer und dem Lipid gemischt werden.

Die so erhaltene vorzugsweise homogene Mischung wird daraufhin auf eine Temperatur erhitzt, welche höher als die Temperatur des Schmelzpunkts des eingesetzten Lipids ist, und anschließend mittels Extrusion, insbesondere mittels Schmelzextrusion, extrudiert.

Gleichzeitig oder nach der Extrusion des Kerns (d. h. des Extrudats ohne eine Überzugsmischung) erfolgt gegebenenfalls das Aufbringen einer vorzugsweise homogenen Überzugmischung bzw. -zusammensetzung, welche aus zumindest einem der oben genannten Komponenten gemäß Schritt (i) der erfindungsgemäßen Depotform besteht. Bevorzugt erfolgt das Aufbringen der Überzugsmischung gleichzeitig mit der Extrusion des Kerns.

Vorteilhaft umfassen dabei die Komponenten gemäß Schritt (i) mindestens etwa 50 Gew.-% des Trockengewichts der erfindungsgemäßen Depotform, bevorzugt mindestens etwa 55 Gew.-%, besonders bevorzugt mindestens etwa 60 Gew.-%, insbesondere bevorzugt mindestens etwa 62 Gew.-%, des Gesamtgewichts der erfindungsgemäßen Depotform.

Insofern es sich bei dem Wirkstoff um einen thermosensitiven Wirkstoff handelt, kann eine Extrusion vorteilhaft bei Temperaturen erfolgen, bei welchen auch thermosensitive Wirkstoffe ohne Beeinträchtigung verarbeitet werden können. In diesem Fall erfolgt die Auswahl des zumindest einen Lipids so, dass dessen Schmelzpunkt vorteilhaft unter der Temperatur liegt, bei welcher der Wirkstoff thermisch beeinträchtigt wird. Dies ist insbesondere für Proteinwirkstoffe und dergleichen von Interesse.

Insofern die Herstellung nicht bereits unter aseptischen Bedingungen stattgefunden hat, kann ein vorteilhaftes Herstellverfahren vor einem möglichen Verpackungsschritt eine Sterilisierung der erfindungsgemäßen extrudierten Depotform vorsehen. Jedoch kann eine erfindungsgemäße Depotform auch ohne einen Sterilisierungsvorgang oder auch unter Bedingungen, welche nicht aseptisch sind, hergestellt werden.

Des Weiteren kann die erfindungsgemäße extrudierte Depotform einem Verpackungsvorgang unterzogen werden, in welchem die Depotform nach einem möglichen Sterilisierungsvorgang direkt in eine Verpackungseinheit verpackt wird. Alternativ kann die extrudierte Depotform auch zunächst in einen zur Applikation der erfindungsgemäßen Depotform vorgesehenen Applikator eingebracht und gemeinsam mit diesem in eine Verpackungseinheit verpackt werden.

Schließlich umfasst die vorliegende Erfindung eine extrudierte Depotform, welche nach einem vorstehend beschriebenen Verfahren erhältlich ist.

Weitere besonders vorteilhafte Ausgestaltungen und Weiterbildungen der Erfindung ergeben sich aus den abhängigen Ansprüchen sowie der nachfolgenden Beschreibung, wobei die Patentansprüche einer bestimmten Kategorie auch gemäß den abhängigen Ansprüchen einer anderen Kategorie weitergebildet sein können und Merkmale verschiedener Ausführungsbeispiele zu neuen Ausführungsbeispielen kombiniert werden können.

Gemäß einer bevorzugten Ausführungsform ist die zumindest eine erste Verbindung aus der Klasse der organischen Polymere auf Basis von Milchsäure und/oder Glykolsäure ausgewählt aus Poly(L-lactid), Poly(D,L-lactid), Poly(glycolid), Poly(L-lactid-co-D,L-lactid), Poly(L-lactid-co-glycolid), Poly(D,L-lactid-co-glycolid), Poly(meso-lactid), Poly(D,L-lactid-co-trimethylencarbonat), Poly(L-lactid-co-meso-lactid), Poly(L-lactid-co-epsilon-caprolacton), Poly(D,L-lactid-co-meso-lactid), Poly(D,L-lactid-co-epsilon-caprolacton), Poly(meso-lactid-co-glycolid), Poly(meso-lactid-co-trimethylencarbonat), Poly(meso-lactid-co-epsilon-caprolacton), Poly(glycolid-co-trimethylencarbonat), Poly(glycolid-co-epsilon-caprolacton) und/oder Poly(glycolid-co-caprolacton).

Als besonders bevorzugte Verbindung/en aus der Klasse der organischen Polymere auf Basis von Milchsäure und/oder Glykolsäure eignen sich dabei Poly(D,L-lactid) (PLA) und Poly(D,L-lactid-co-glycolid) (PLGA), welche beispielsweise bei der Firma Evonik Industries AG (Deutschland) unter den Bezeichnungen R 202 H (Poly(D,L-lactid)) bzw. RG 502 H und RG 752 H (PLGA) erhältlich sind.

Unter der Bezeichnung Polylactid (*syn.* Polymilchsäuren, PLA) sind vorliegend Polymere der Milchsäure zu verstehen. Die aufgrund des asymmetrischen Kohlenstoffatoms optisch aktiven Polymere treten in der Form von D- oder L-Lactiden auf.

Unter der Bezeichnung "Polyglycolid" (PGA) sind vorliegend Polymere der Glycolsäure (*syn.* Hydroxyessigsäure) zu verstehen.

Unter der Bezeichnung Poly(lactid-co-glycolid) (PLGA) ist ein Copolymer aus den Monomeren Lactid und Glycolid zu verstehen, welche in unterschiedlichen Verhältnissen eingesetzt werden können. Hierbei bildet sich ein Polyester aus D,L-Milchsäure und Glycolsäure, welcher bioabbaubar ist.

Das Molekulargewicht des organischen Polymers auf Basis von Milchsäure und/oder Glykolsäure, insbesondere von PLA bzw. PLGA, kann prinzipiell in einem breiten Bereich variieren. Bevorzugt liegt das Molekulargewicht jedoch bei mindestens etwa 5 kDa und/oder bei höchstens etwa 100 kDa. Besonders bevorzugt liegt das Molekulargewicht bei mindestens etwa 7 kDa und/oder bei höchstens etwa 60 kDa, insbesondere bei mindestens etwa 9 kDa und/oder bei höchstens etwa 40 kDa, insbesondere bevorzugt bei mindestens etwa 10 kDa und/oder bei höchstens etwa 30 kDa.

Gemäß einer bevorzugten Ausführungsform weist das in der extrudierten Depotform enthaltene Lipid einen Schmelzpunkt von mindestens etwa 60 °C und/oder von höchstens etwa 80 °C auf. Besonders bevorzugt liegt der Schmelzpunkt des Lipids bei mindestens etwa 62 °C, insbesondere bei mindestens etwa 69 °C. Höchstens liegt der besonders bevorzugte Schmelzpunkt bei etwa 77 °C, insbesondere bei etwa 73 °C.

Gemäß einer weiter bevorzugten Ausführungsform ist das zumindest eine Lipid ausgewählt aus Mono-, Di- und/oder Triglyceriden, beispielsweise Veresterungen von Glycerin mit gesättigten und/oder ungesättigten Fettsäuren einer Länge von mindestens 5 und/oder höchstens 26 Kohlenstoffatomen, Phosphatidsäure, Lecithin, Phosphatidylethanolamin, Phosphatidylinositol, Phosphatidylserin, Diphosphatidylglycerin, Ceramiden, Cerebrosiden, Gangliosiden, Sphingophospholipiden, Sphingomyelinen, Sphingosulfatiden, Glycosphingosiden, Acylaminozuckern, Acylaminozuckerglykanen, Acyltrehalosen, Acyltrehaloseglykanen, Sorbitanfettsäureestern, Squalen, Steroiden, Polyketiden, Sterollipiden, Prenollipiden, Cholesterol, Hartfetten, Wachsen, und Salzen und Derivaten davon.

Beispiele für bevorzugte Lipide sind Hartfette, welche beispielsweise aus einem Gemisch von Mono-, Di- und Triglyceriden bestehen, das durch Veresterungen von Fettsäuren natürlichen Ursprungs mit Glycerin oder durch Umesterung von Fetten natürlichen Ursprungs erhalten werden kann. Besonders bevorzugte Lipide umfassen Fettsäuren mit einer Anzahl an C-Atomen von mindestens 12 und/oder höchstens 22, insbesondere von mindestens 18 und/oder höchstens 22. Ein ganz besonders bevorzugtes Lipid ist ausgewählt aus Glyceryltristearat. Solche Hartfette sind in der Pharmacopoea Europaea (Ph. Eur. 8. Ausgabe, Grundwerk 2014) beschrieben und können beispielsweise unter der Bezeichnung Dynasan 112, Dynasan 116 und/oder Dynasan 118 ausgewählt sein. Diese sind beispielsweise bei der Firma IOI Oleo GmbH (Deutschland) erhältlich. Insofern thermosensitive Wirkstoffe zur Anwendung kommen sollen, eignen sich vorzugsweise Lipide mit einem Schmelzpunkt unter 50 °C, wie Witepsol E85, Witepsol H5, Witepsol H12, Witepsol H37 und/oder Witepsol H15, welche beispielsweise von der Firma IOI Oleo GmbH (Deutschland) bezogen werden können.

Ferner können extrudierte Depotformen auch mehr als eine Verbindung aus der Klasse der Lipide umfassen. Bevorzugt umfasst die extrudierte Depotformen jedoch lediglich eine Verbindung aus der Klasse der Lipide.

Gemäß einer bevorzugten Ausführungsform liegt das Verhältnis der zumindest einen ersten Verbindungen aus der Klasse der Polymere und der zumindest einen zweiten Verbindung aus der Klasse der Lipide bei höchstens etwa 25:1, bevorzugt bei höchstens etwa 20:1, besonders bevorzugt bei höchstens etwa 19:1. Das Verhältnis der zumindest einen ersten Verbindungen aus der Klasse der Polymere und der zumindest einen zweiten Verbindung aus der Klasse der Lipide liegt jedoch mindestens etwa 5:1, bevorzugt bei mindestens etwa 10:1, besonders bevorzugt bei mindestens etwa 12:1.

Beispielhafte Kombinationen von zumindest einer Verbindung aus der Klasse der organischen Polymere auf Basis von Milchsäure und/oder Glykolsäure und zumindest einer Verbindung aus der Klasse der Lipide sind in Tabelle 1 zusammengefasst.

**Tabelle 1: Beispielhafte Zusammensetzungen erfindungsgemäßer Depotformen.**

| **Organisches Polymer auf Basis von Milchsäure und/oder Glykolsäure** | **Lipid** | **Gewichtsverhältnis** |
|---|---|---|
| 31,5 Gew.-% Polymilchsäure-co-glykolsäure Polymer und 31.5 Gew.-% Polymilchsäure | 3,3 Gew.-% Triglycerid | 19:1 |
| 30,0 Gew.-% Polymilchsäure-co-glykolsäure Polymer und 30,0 Gew.-% Polymilchsäure | 3,5 Gew.-% Triglycerid | 17:1 |
| 57,0 Gew.-% Polymilchsäure-co-glykolsäure Polymer | 10,0 Gew.-% Glycerol dibehenate | 6:1 |
| 28,5 Gew.-% Polymilchsäure-co-glykolsäure Polymer und 28,5 Gew.-% Polymilchsäure | 3,0 Gew.-% Triglycerid | 19:1 |
| 38,0 Gew.-% Polymilchsäure-co-glykolsäure Polymer und 26,0 Gew.-% Polymilchsäure | 3,0 Gew.-% Triglycerid | 21:1 |

In den erfindungsgemäßen Depotformen ist zumindest ein Wirkstoff enthalten. Dieser zumindest eine Wirkstoff kann, ohne beschränkend zu sein, ausgewählt sein aus der Klasse der Antibiotika, Antimikrobiotika, Antimykotika, Antiseptika, Chemotherapeutika, Zytostatika, Metastaseinhibitoren, Antiallergika, Antikoagulanzien, Sexualhormone, Sexualhormoninhibitoren, Hämostyptika, Hormone, Peptidhormone, Fusionsproteine, Antidepressiva, Impfstoffe, Gonadotropin-Releasing-Hormon-Analoga, Wachstumsfaktorinhibitoren, Hormonmimetika, Multiple Sklerose-Therapeutika, Programmed Cell Death Rezeptor 1-Antagonisten, Neuroleptika, Komplementsysteminhibitoren, Vitamine, Antihistamine, Antikörper, Antikörperfragmente, Nucleinsäuren, DNA, Plasmid-DNA, kationischen DNA-Komplexen und RNA, siRNA, mRNA und Antidiabetika.

Nützliche Wirkstoffe umfassen, ohne beschränkend zu sein, Heparin, Heparinderivate, Hirudin, Acetylsalicylsäure, Enoxaparin, Liraglutid, Albiglutid, Dulaglutid, Lixisenatid, Exenatid, Insulin, Insulinanaloga, Acarbose, Glatirameracetat, Octreotid, Pasireotid, Lanreotid und/oder Valpreotid, Desmopressin, Oxytocin, Zafirlukast, Buserelin, Somatostatin, Glibenclamid, Gliclazid, Glimepirid, Gliquidon, Pioglitazon, Miglitol, Nateglinid, Mitiglinid, Repaglinid, Sitagliptin, Vildagliptin, Dexamethason, Prednisolon, Corticosteron, Budesonid, Östrogen, Sulfasalazin, Mesalazin, Risperidon, Paclitaxel, 5-Fluoruracil, Cisplatin, Vinblastin, Vincristin, Epothilone, Endostatin, Angiostatin, D-Phe-Pro-Arg-Chlormethylketon, Aflibercept und monoklonale Antikörper wie beispielsweise Adalimumab, Aducanumab, Benralizumab, Bevacizumab, Certolizumab, Denosumab, Dupilumab, Efalizumab, Erenumab, Infliximab, Ipilimumab, Mepolizumab, Natalizumab, Nemolizumab, Ocrelizumab, Omalizumab, Pembrolizumab, Pertuzumab, Ranibizumab, Reslizumab, Rituximab, Solanezumab, Tocilizumab, Tralokinumab, Trastuzumab, Ustekinumab und Vedolizumab.

Bevorzugte extrudierte Depotformen enthaltend zumindest einen Wirkstoff können dabei zur Behandlung der Akromegalie; von Symptomen, die mit (funktionell aktiven) gastroenteropankreatischen endokrinen Tumoren assoziiert sind, wie beispielsweise Karzinoide mit Merkmalen des Karzinoidsyndroms; fortgeschrittenen neuroendokrinen Tumoren; und Thyreoidea-stimulierendes Hormon (TSH)-sezernierende Hypophysenadenome; Krebserkrankungen wie beispielsweise multiples Myelom, Mantelzelllymphom, diffuses großzelliges B-Zell-Lymphom, akutes myeloides Lymphom, follikulares Lymphom, chronische lymphozytische Leukämie, Brust-, Lungen-, Endometrium-, Eierstock-, Magen-, Gebärmutterhals- oder Prostatakrebs, Pankreaskarzinom, Glioblastom, Nierenkarzinom; hepatozelluläres Karzinom, Dickdarmkarzinom, neuroendokrine Tumore, Kopf-und-Nacken Tumore, Sarkoma; Tumorsyndromen resultierend direkt oder indirekt aus genetischen Defekten in Tumorsuppressorgenen wie beispielsweise P53, PTEN oder VHL, endometriales Karzinom, Lymphangioleiomyomatose, Neurofibromatose 1, von Hippel-Lindau Erkrankung; sowie von rheumatoider Arthritis; Spondylitis ankylosans (Morbus Bechterew); Psoriasis-Arthritis; Schuppenflechte; Osteoarthritis; Gicht; Asthma; Bronchitis; allergischer Rhinitis; chronisch obstruktiver Lungenerkrankung; zystischer Fibrose; chronisch entzündlichen Darmerkrankungen wie Reizdarm, muköser Kolitis, ulzerativer Kolitis, Morbus Crohn, Chorea Huntington; Gastritis; Ösophagitis; Hepatitis; Pankreatitis; Nephritis; Lupus erythematodes; Atherosklerose; Restenose in Folge von Angioplasie; linksventrikulärer Hypertrophie; Myokardialinfarkt; Schlaganfall; ischämischen Schäden von Herz, Lunge, Darm, Nieren, Leber, Pankreas, Milz und Gehirn; akuter oder chronischer Organtransplantat-Abstoßung; Makuladegeneration; des diabetischen Makulaödems; Hyposomatotropismus; Anämie; Fruchtbarkeitsstörungen; Fettleibigkeit; Pubertas praecox; Endometriose; Mastodynie; Tourette-Syndrom; Depressionen; Persönlichkeitsstörungen; Zwangserkrankungen; ADHS bei Kindern; Reizbarkeit bei Fetalem Alkoholsyndrom und Autismus; Wahnvorstellungen; Halluzinationen; Epilepsie; Alzheimer Erkrankung; Parkinson Erkrankung; der paroxysmalen nächtlichen Hämoglobinurie; als Beruhigungsmittel; zu geschlechtsangleichenden Maßnahmen; Multipler Sklerose und Diabetes eingesetzt werden.

Gemäß einer bevorzugten Ausführungsform enthält eine vorteilhafte extrudierte Depotform zumindest einen Wirkstoff aus der Klasse der Peptidhormone. Besonders bevorzugt ist der zumindest eine Wirkstoff ausgewählt aus Octreotid, Pasireotid, Lanreotid und/oder Valpreotid. Insbesondere ist der zumindest eine Wirkstoff ausgewählt aus Octreotid.

Weiter bevorzugt ist der zumindest eine Wirkstoff ausgewählt aus Nucleinsäuren, bevorzugt aus DNA, Plasmid-DNA, kationischen DNA-Komplexen und RNA, siRNA und mRNA. Besonders bevorzugt ist der zumindest eine Wirkstoff ausgewählt aus RNA, siRNA und mRNA, insbesondere aus selbstreplizierender RNA oder mRNA.

Eine solche selbstreplizierende RNA ist aus dem Stand der Technik bekannt und kann beispielsweise aus Viren gewonnen werden.

Eine bevorzugte (selbstreplizierende) RNA oder mRNA kann vorzugsweise als Vakzine und/oder zur Krebstherapie eingesetzt werden. Die RNA oder mRNA codiert hierbei für ein gewünschtes Antigen, etwa von einem Krankheitserreger. Die Impfstoffe stimulieren so beispielsweise auch Immunreaktionen gegen Tumorassoziierte Antigene und regen Abwehrzellen an, die Krebszellen zu bekämpfen.

Ein weiteres Einsatzgebiet ist die Verwendung von RNA und/oder mRNA als Wirkstoff in der vorteilhaften Depotform zur Bildung von körpereigenen Proteinen und/oder Enzymen. So können nicht funktionale körpereigene Proteine und Enzyme mittels RNA und/oder mRNA, welche für ein entsprechendes funktionales Protein bzw. Enzym codieren, durch diese RNA bzw. mRNA-codierten funktionalen Proteine bzw. Enzyme ersetzt werden.

Bevorzugt ist die Abgabe von RNA bzw. mRNA in oder an ein spezifisches Organ oder Gewebe von Relevanz. Insbesondere erfolgt die Abgabe von RNA und/oder mRNA aus der Depotform in das Auge. Besonders bevorzugt erfolgt eine solche Anwendung zur Therapie der Makuladegeneration und/oder des Glaukoms, von Diabetes bedingten Augenerkrankungen sowie onkologisch bedingten Augenerkrankungen.

Vorteilhaft können die immunstimulierenden bzw. therapeutischen Eigenschaften bzw. einer Wirkung der RNA bzw. mRNA durch die Zugabe eines Adjuvans erhöht werden. Solche Adjuvantien sind im Stand der Technik bekannt. So zeigte sich, dass beispielsweise die sich selbst vervielfältigende RNA-Vakzine wirksamer ist, wenn sie in eine kationische Öl-in-Wasser Nanoemulsion basierend auf Squalen und Polysorbate (Tween 80) und Sorbitantrioleat (Span 85), Natriumcitrat und Citronensäure, wie beispielsweise Adjuvans MF59 (Novartis), formuliert ist. Eine Wirkverstärkung bei mRNA lässt sich beispielsweise erreichen, indem die Moleküle mit TriMix (mRNAs, welche drei das Immunsystem aktivierenden Proteinen caTLR4, CD40L und CD70 codieren), zusammengebracht werden.

Insbesondere werden bevorzugte Depotformen eingesetzt für die Behandlung der Akromegalie; von Symptomen, die mit (funktionell aktiven) gastroenteropankreatischen endokrinen Tumoren assoziiert sind, wie beispielsweise Karzinoide mit Merkmalen des Karzinoidsyndroms; fortgeschrittenen neuroendokrinen Tumoren; und Thyreoidea-stimulierendes Hormon (TSH)-sezernierende Hypophysenadenome.

Der insbesondere bevorzugte Wirkstoff Octreotid kann dabei vorzugsweise ein Polypeptid aus Aminosäuren mit der folgenden Sequenz umfassen:
FCFWKTCT (Phe-Cys-Phe-Trp-Lys-Thr-Cys-Thr)

Der zumindest eine Wirkstoff kann ferner in verschiedenen Formen in der Depotform enthalten sein, je nachdem, welche Form die optimale Abgabeeigenschaft des Wirkstoffs aus der Depotform ergibt. Aminosäurebasierte Wirkstoffe können im Allgemeinen als Cyclopeptid, Oligopeptid oder Polypeptid oder andere pharmakologisch akzeptable Derivate oder als Komponenten von molekularen Komplexen vorliegen. Dabei können die Aminosäuren sowohl über α-Peptidbindungen als auch über ω-Peptidbindungen miteinander verknüpft sein. Der zumindest eine Wirkstoff kann auch als Salz wie beispielsweise als Acetat, oder auch in Form der freien Base bzw. Säure vorliegen.

Des Weiteren kann zumindest eine der Aminosäuren der vorstehend als bevorzugte Wirkstoffe genannten aminosäurebasierten Wirkstoffe posttranslationale Modifikationen aufweisen. Hierbei beeinflussen diese posttranslationalen Modifikationen vorteilhaft die Eigenschaften des Wirkstoffs, insbesondere hinsichtlich der Freisetzung und Wirkung, nicht.

Prinzipiell kann der Gehalt des Wirkstoffs in der erfindungsgemäßen Depotform in einem breiten Bereich variieren. Eine vorteilhafte Wirkstoffmenge, bevorzugt eines Peptidhormons, besonders bevorzugt Octreotid, Pasireotid, Lanreotid und/oder Valpreotid, insbesondere Octreotid, liegt bei etwa 0,3 Gew.-% bis etwa 50 Gew.-%, vorzugsweise bei etwa 3 Gew.-% bis etwa 45 Gew.-%, besonders bevorzugt bei etwa 4 Gew.-% bis etwa 40 Gew.-%, insbesondere bei etwa 7 Gew.-% bis etwa 35 Gew.-%.

Für eine effektive Therapie ist die Wirkstoffkonzentration des aktiven Wirkstoffs, welche nach Verabreichung einer Depotform im Blut des Anwenders herrscht, von Bedeutung. Vorteilhaft weist daher eine bevorzugte extrudierte Depotform, welche vorzugsweise ein Peptidhormon, insbesondere Octreotid, enthält, eine *in vitro* Wirkstofffreisetzung nach 7 Tagen von mindestens etwa 20 Gew.-% und/oder nach 28 Tagen von mindestens etwa 60 Gew.-% auf, bezogen auf die gesamte im Implantat enthaltene Wirkstoffmenge.

Erfindungsgemäße extrudierte Depotformen sind ferner für kosmetische Anwendungen geeignet. Insbesondere kann eine vorteilhafte Depotform für die kosmetische Faltenglättung eingesetzt werden. Dabei wird die erfindungsgemäße Zusammensetzung zur lokalen, insbesondere zur gezielten Faltenglättung, eingesetzt, insbesondere bevorzugt zur Vermeidung von Falten, zur Hautstraffung und zum Schutz vor Hautalterung. Beispielhafte Wirkstoffe hierfür können ausgewählt sein aus Hyaluronsäure, Kollagen und/oder Botox.

Vorteilhafte Depotformen können überdies zumindest einen für subkutane Applikationsformen gebräuchlichen Hilfsstoff enthalten, welcher die Wirkstofffreisetzung aus der Depotform, die Wirkstoffstabilität, die Plasmahalbwertszeit und/oder die Bioverfügbarkeit des zumindest einen Wirkstoffs beeinflussen kann. Vorteilhaft unterstützt ein bevorzugter Hilfsstoff die kontrollierte Abgabe des zumindest einen Wirkstoffs aus der Depotform. Insbesondere trägt ein solcher Hilfsstoff zu einer langanhaltenden Wirkstoffabgabe bei, ohne jedoch die Biokompatibilität nachteilig zu beeinflussen. Alternativ oder zusätzlich kann die Zugabe eines solchen Hilfsstoffs die Stabilität des in der Depotform enthaltenen Wirkstoffs verbessern. Dies ist von besonderem Interesse, wenn die Depotform für eine langanhaltende Applikation von mehreren Wochen bis zu einem Jahr vorgesehen ist.

Hierbei zu nennen sind vor allem Substanzen, die bei der Herstellung von subkutanen Implantaten eingesetzt werden und physiologisch unbedenklich sind. Vorzugsweise weisen die Hilfsstoffe eine hohe Biokompatibilität auf, sodass die Hilfsstoffe und jegliche Abbauprodukte der Hilfsstoffe nicht toxisch für den Anwender sind und keine unerwünschten Nebenwirkungen hervorrufen.

Es hat sich gezeigt, dass die Zugabe von Porenbildnern vorteilhaft die Abgabe des zumindest einen Wirkstoffs aus der subkutanen Depotform verbessern kann. Ein solcher Porenbildner kann beispielsweise aus der Gruppe der hydrophilen Stoffe wie Calciumsulfat, Calciumhydrogenphosphat, Zucker wie beispielsweise Glucose, Lactose, Fructose, Mannitol, Trehalose, Dextrine, Maltodextrin, Saccharose, Sorbit, Xylit, Stärke bzw. deren Derivaten wie beispielsweise Hydroxyethylstärke, Polyvinylpyrrolidon, Polyethylenglycol (PEG) wie beispielsweise PEG 6000 oder PEG 8000, Natriumchlorid, Natriumcitrat, Zitronensäure, Hyaluronsäuren, Polyvinylalkohol, Polyacrylsäure und deren Derivaten, Polymethacrylsäure und deren Derivaten, Polymethylmethacrylat, Polystyrol, Copolymere mit Monomeren von Methylmethacrylat und Styrol und Mischungen davon ausgewählt sein.

Als besonders bevorzugte Porenbildner eignen sich Trehalose und/oder Hydroxyethylstärke und/oder Polyethylenglycol, welche beispielsweise von der Firma Clariant oder Sigma-Aldrich (Österreich) bezogen werden können.

Das Molekulargewicht eines Porenbildners, insbesondere von PEG, liegt dabei bevorzugt bei mindestens etwa 1 kDa, besonders bevorzugt bei mindestens etwa 3 kDa, insbesondere bei mindestens etwa 4 kDa. Höchstens liegt ein bevorzugtes Molekulargewicht von PEG bei etwa 10 kDa, besonders bevorzugt bei höchstens etwa 8 kDa.

Gemäß einer weiter bevorzugten Ausführungsform kann eine vorteilhafte Depotform Hilfsstoffe umfassen, welche eine Acylierung von Polypeptiden, vorzugsweise von Octreotid, Pasireotid, Lanreotid und/oder Valpreotid, besonderes bevorzugt von Octreotid, inhibieren. Hierfür eignen sich bevorzugt divalente Salze bzw. Salze von divalenten Metallionen, wie beispielsweise Calciumchlorid.

Gemäß einer weiter bevorzugten Ausführungsform umfasst ein Hilfsstoff zumindest ein Enzym. Ein solches Enzym kann die Abgabe des zumindest einen Wirkstoffs aus der vorteilhaften Depotform günstig beeinflussen, ohne hierbei nachteilig auf die Stabilität und/oder die Bioabbaubarkeit der Depotform zu wirken. Vorteilhaft bewirkt ein solches Enzym eine verbesserte Bioabbaubarkeit der Depotform. Besonders bevorzugt ist ein solches Enzym ausgewählt aus der Gruppe von Lipasen. Eine solche Lipase kann beispielsweise über die Firma Sigma-Aldrich bezogen werden.

Die Wirkstoffabgaberate kann ferner durch Zugabe eines quellbaren Polymers gesteigert werden, welches bevorzugt ausgewählt ist aus Kollagen, Gelatine und deren Derivaten, Stärke und deren Derivaten (bevorzugt Hydroxyethylstärke, Hydroxypropylstärke, Carboxymethylstärke), Cellulosederivaten, Chitin, Chitosan und deren Derivaten, Polyamiden, Polyhydroxysäuren, Polyhydroxybutyraten, Polyhydroxyvaleraten, Polycaprolactonen und Polydioxanonen. Dies ist insbesondere für Wirkstoffe, für welche eine höhere Dosis benötigt wird und/oder für wirkstoffhaltige Depotformen mit einer kürzeren Applikationsdauer wie beispielsweise einer Applikationsdauer von wenigen Wochen bis zu wenigen Monaten, relevant. Ein besonders bevorzugtes quellbares Polymer ist dabei Hydroxyethylstärke (HES) und kann bei Sigma Aldrich (Österreich) bezogen werden.

Vorzugsweise liegt das Molekulargewicht eines quellbaren Polymers, insbesondere von HES, bei mindestens etwa 50 kDa, bevorzugt bei mindestens etwa 70 kDa, insbesondere bei mindestens etwa 100 kDa, insbesondere bevorzugt bei mindestens etwa 120 kDa. Höchstens liegt das Molekulargewicht eines quellbaren Polymers, insbesondere von HES, bei etwa 400 kDa, besonders bevorzugt bei höchstens etwa 300 kDa, insbesondere bei höchstens etwa 200 kDa, insbesondere bevorzugt bei höchstens etwa 150 kDa.

Der Substitutionsgrad von HES, also das Verhältnis der Anzahl der mit Hydroxyalkylgruppen modifizierten Glucoseeinheiten zur Gesamtzahl der Monomereinheiten, liegt bei mindestens etwa 0,1, bevorzugt bei mindestens etwa 0,2, insbesondere bei mindestens etwa 0,3. Höchstens liegt der Substitutionsgrad bei etwa 1, bevorzugt bei etwa 0,7, insbesondere bei etwa 0,5.

Zusätzlich können die erfindungsgemäßen Depotformen noch weitere übliche, dem Fachmann bekannte Hilfsstoffe enthalten, wie beispielsweise Tocopherole, z. B. α-Tocopherol, β-Tocopherol, γ-Tocopherol, δ-Tocopherol und Mischungen davon (Vitamin E), welche insbesondere als Antioxidantien eingesetzt werden.

In einer vorteilhaften Ausführungsform inaktiviert ein solches Antioxidans reaktive Sauerstoffspezies in der Depotform, wodurch eine Oxidation des Wirkstoffs verlangsamt oder gänzlich verhindert wird, und bewirkt damit eine verbesserte Stabilität des Wirkstoffs und somit eine längere Haltbarkeit der erfindungsgemäßen Depotform sowohl während der Lagerung als auch während der Anwendung.

Vorteilhaft bewirkt ein Gehalt eines oder mehrerer der bevorzugten Hilfsstoffe eine kontrollierte und anhaltende Wirkstoffabgabe aus der bevorzugten extrudierten Depotform.

Vorteilhafte Zusammensetzungen einer extrudierten Depotform sind wie folgt dargestellt:
(i) 0,1 bis 50 Gew.-% Wirkstoff, bevorzugt 1 bis 50 Gew.-% Wirkstoff, insbesondere 2,5 bis 40 Gew.-% Wirkstoff,
(ii) 1 bis 95 Gew.-% eines organischen Polymers auf Basis von Milchsäure und/oder Glykolsäure, bevorzugt 10 bis 89 Gew.-% eines organischen Polymers auf Basis von Milchsäure und/oder Glykolsäure,
(iii) 1 bis 89 Gew.-% eines Lipids, bevorzugt 1 bis 60 Gew.-% eines Lipids,
(iv) gegebenenfalls 1 bis 25 Gew.-% Trehalose, bevorzugt 5 bis 20 Gew.-% Trehalose,
(v) gegebenenfalls 1 bis 25 Gew.-% PEG, bevorzugt 5 bis 20 Gew.-% PEG.

Besonders bevorzugt umfasst eine Zusammensetzung einer extrudierten Depotform
(i) 5 bis 45 Gew.-% Wirkstoff, insbesondere 10 bis 40 Gew.-% Wirkstoff,
(ii) 30 bis 85 Gew.-% eines organischen Polymers auf Basis von Milchsäure und/oder Glykolsäure, vorzugsweise PLGA und/oder PLA
(iii) 1 bis 30 Gew.-% eines Lipids,
(iv) gegebenenfalls 2,5 bis 18 Gew.-% Trehalose, bevorzugt 3 bis 16 Gew.-% Trehalose, insbesondere 3,5 bis 15 Gew.-% Trehalose, und
(v) gegebenenfalls 3 bis 17,5 Gew.-% PEG.

Insbesondere bevorzugt sind die Bestandteile einer extrudierten Depotform ausgewählt aus
(i) 15 bis 35 Gew.-% Wirkstoff,
(ii) 40 bis 75 Gew.-% eines organischen Polymers auf Basis von Milchsäure und/oder Glykolsäure, vorzugsweise PLGA und/oder PLA
(iii) 1 bis 10 Gew.-% eines Lipids,
(iv) gegebenenfalls 5 bis 10 Gew.-% Trehalose, und
(v) gegebenenfalls 5 bis 15 Gew.-% PEG.

Weiter bevorzugt sind die Bestandteile einer extrudierten Depotform ausgewählt aus
(i) 0,1 bis 50 Gew.-% Wirkstoff, bevorzugt 1 bis 50 Gew.-% Wirkstoff, insbesondere 2,5 bis 40 Gew.-% Wirkstoff, insbesondere bevorzugt 5 bis 20 Gew.-% Wirkstoff,
(ii) 40 bis 75 Gew.-% eines organischen Polymers auf Basis von Milchsäure und/oder Glykolsäure, vorzugsweise PLGA und/oder PLA
(iii) 1 bis 10 Gew.-% eines Lipids,
(iv) 0,1 bis 2 Gew.-% einer Lipase, bevorzugt 0,2 bis 1 Gew.-%,
(v) gegebenenfalls 5 bis 10 Gew.-% Trehalose, und
(vi) gegebenenfalls 5 bis 15 Gew.-% PEG.

Insbesondere liegt ein vorteilhafter Wirkstoffgehalt bei etwa 30 bis 33 Gew.-%, ein Gehalt eines bioabbaubaren organischen Polymers liegt bei etwa 50 bis 70 Gew.-% und ein Gehalt eines Lipids liegt bei etwa 5 Gew.-%.

Gemäß einer weiteren bevorzugten Ausführungsform kann Trehalose ganz oder teilweise durch HES ersetzt werden.

Wie eingangs erwähnt, werden erfindungsgemäße extrudierte Depotformen mittels Extrusion hergestellt. Es hat sich gezeigt, dass sich dabei die Eigenschaften der zur Extrusion vorbereiteten Mischung der zumindest zwei Verbindungen aus der Klasse der organischen Polymere auf Basis von Milchsäure und/oder Glykolsäure und aus der Klasse der Lipide mit dem zumindest einen Wirkstoff verbessert, wenn der Wirkstoff als getrocknetes Pulver, bevorzugt als sprüh- oder gefriergetrocknetes Pulver bzw. Lyophilisat, den zumindest zwei Verbindungen beigemischt wird.

Insofern Wirkstoffe in gelöstem Zustand bezogen werden, erfolgt vor dem Mischen der Substanzen zur Herstellung der erfindungsgemäßen Depotform bevorzugt ein Trocknungsschritt, insbesondere ein Lyophilisierungsschritt.

Hierfür können dem Wirkstoff prinzipiell eine Vielzahl von Substanzen zugegeben werden, um vorteilhaft die Erhaltung der Bioaktivität des Wirkstoffs zu gewährleisten. Solche Substanzen werden auch als Kryo- bzw. Lyoprotektoren bezeichnet, wobei Lyoprotektoren in diesem Zusammenhang dem Schutz der Substanzen bei der Trocknung dienen und Kryoprotektoren eine entsprechende Aufgabe während des Gefrierens haben.

Ferner hat sich herausgestellt, dass sich die Länge der Wirkstofffreisetzung nach der subkutanen Applikation beeinflussen lassen kann, indem die Extrudate direkt nach der Extrusion nicht auf Umgebungstemperatur abgekühlt, sondern beispielsweise in einem Trockenschrank oder Inkubator für eine gewisse Zeit bei erhöhter Temperatur gelagert werden. Hierfür eignet sich vorzugsweise eine Zeitdauer im Bereich von etwa 0,5 bis etwa 5 Stunden. Die optionale Lagerung bei erhöhter Temperatur, auch als Temperschritt bezeichnet, richtet sich dabei im Wesentlichen nach dem Schmelzpunkt des zumindest einen Lipids und liegt beispielsweise im Bereich von etwa 40 bis etwa 80 °C, bevorzugt bei etwa 55 bis etwa 75 °C, besonders bevorzugt bei etwa 65 bis 70 °C. Selbstverständlich richtet sich dabei eine bevorzugte Lagerungstemperatur stets auch nach der Temperaturstabilität des zumindest einen Wirkstoffs.

Bevorzugte Depotformen können überdies durch ein Rundungsverfahren, insbesondere durch Sphäronisation, hergestellt werden. Dabei wird das zylinderförmige Extrudat vorteilhaft so abgerundet, dass evtl. bei der Extrusion entstandene Formen wie beispielsweise Ecken und Kanten, welche die Applikationseigenschaften nachteilig beeinflussen können, entfernt werden. Die Sphäronisation kann darüber hinaus zur Herstellung von Mikropartikeln eingesetzt werden, welche im Anschluss subkutan verabreicht werden und somit ebenfalls eine bioabbaubare Depotform darstellen.

Vorteilhaft weist eine Depotform einen homogenen Überzug auf, der aus zumindest einer auf dem Kern aufgebrachten Schicht besteht und vorzugsweise den initialen Freisetzungsschub von Wirkstoff aus der Depotform begrenzt und therapeutische Konzentrationen des zumindest einen Wirkstoffs über einen anhaltenden Zeitraum gewährleistet. Ein bevorzugter Überzug weist eine Mischung von Substanzen bzw. eine Zusammensetzung auf, welche aus zumindest einer der oben genannten Komponenten a) bis c) der erfindungsgemäßen Depotform ausgewählt sind bzw. ist, und kann somit beispielsweise auch wirkstofffrei ausgestaltet sein. Insofern der Überzug einen Wirkstoff enthält, kann dessen Gehalt gleich oder unterschiedlich vom Wirkstoffgehalt des Kerns sein. In einer besonders bevorzugten Ausführungsform ist der Überzug jedoch wirkstofffrei ausgestaltet.

Grundsätzlich bewegt sich ein geeignetes Gewicht einer extrudierten Depotform in einem für subkutane Implantate üblichen Bereich. Dabei ist das Gewicht der extrudierten Depotform auch abhängig von der gewünschten Applikationszeit und/oder des Applikationsorts. Ein bevorzugtes Gewicht einer extrudierten Depotform liegt jedoch im Bereich von 1 bis 1000 mg, besonders bevorzugt bei mindestens etwa 20 mg und/oder höchstens etwa 180 mg, insbesondere bei mindestens etwa 50 mg und/oder bei höchstens etwa 150 mg, insbesondere bevorzugt bei mindestens etwa 80 mg und/oder bei höchstens etwa 120 mg.

Eine Depotform im Sinne der Erfindung kann dabei als Stäbchen, Kugel, Würfel, Ellipsoid, Quader, Kissen, Zylinder, Tablette, Pellet, Plättchen oder Brikett ausgestaltet sein.

Erfindungsgemäße Depotformen weisen bevorzugt eine injizierbare Größe auf, können jedoch, falls erwünscht, auch mittels eines chirurgischen Eingriffs an der Verabreichungsstelle eingebracht werden. Dabei haben bevorzugte Depotformen einen Durchmesser von zumindest 0,1 bis 10 mm und eine Länge von zumindest 0,15 bis 50 mm. Insbesondere haben Depotformen einen Durchmesser von zumindest 0,15 bis 7,5 mm und eine Länge von zumindest 0,2 bis 45 mm, insbesondere bevorzugt einen Durchmesser von zumindest 0,2 bis 5 mm und eine Länge von zumindest 0,3 bis 40 mm.

Der Begriff "Durchmesser" bezieht sich dabei auf die längste orthogonal zu einer Rotationsachse verlaufende Strecke, die zwei Punkte des Rands des betreffenden Körpers miteinander verbindet. Als Rotationsachse wird diejenige Gerade bezeichnet, um die man einen Rotationskörper drehen kann.

Der Begriff "Länge" betrifft den Teil der Rotationsachse, der sich innerhalb des Rotationskörpers befindet.

Das Verhältnis von Durchmesser zu Länge bevorzugter Depotformen liegt vorteilhaft im Bereich von 1:30 bis 10:1, bevorzugt im Bereich von 1:15 bis 5:1, insbesondere bevorzugt im Bereich von 1:13 bis 1:1.

Insofern extrudierte Depotformen als Mikropartikel vorliegen, kann der Durchmesser der runden bzw. nahezu runden Partikel bei etwa 1 bis etwa 100 µm liegen, bevorzugt bei etwa 5 bis etwa 90 µm, insbesondere bevorzugt bei etwa 10 bis etwa 80 µm.

Wie bereits oben erläutert, umfasst ein erfindungsgemäßes Verfahren zur Herstellung der extrudierten Depotform das Mischen (a) des zumindest einen Wirkstoffs, (b) der zumindest einen Verbindung aus der Klasse der organischen Polymere auf Basis von Milchsäure und/oder Glykolsäure und (c) der zumindest einen Verbindung aus der Klasse der Lipide, wodurch eine homogene Pulvermischung erhalten wird.

Unter einer Pulvermischung im Sinne der vorliegenden Erfindung wird hierbei ein Gemisch mehrerer fester Bestandteile geeigneter Größe verstanden, wobei die Bestandteile Partikel einer Größe von unter 1 nm aufweisen können. Ferner kann eine Pulvermischung auch Partikel mit einer Größe im Bereich von 1 nm bis 1 µm und/oder Partikel mit einer Größe von über 1 µm aufweisen. Insofern zumindest einer der zu mischenden Bestandteile vor dem Mischen nicht in fester Form vorliegt, kann dieser vor Erstellen der Pulvermischung in den festen Zustand überführt werden, beispielsweise durch Sprühtrocknung oder Gefriertrocknung.

Zur Applikation der erfindungsgemäßen extrudierten Depotform in das subkutane Gewebe können prinzipiell alle dem Fachmann bekannten Applikationsgeräte eingesetzt werden. So können erfindungsgemäße Depotformen beispielsweise durch Spritzen, Kanülen, Applikatoren und Injektoren, insbesondere durch Applikatoren, verabreicht werden.

Schließlich betrifft die vorliegende Erfindung auch ein Kit umfassend eine erfindungsgemäße extrudierte Depotform und einen zur Applikation geeigneten Applikator, mit welchem die Depotform subkutan verabreicht werden kann. Dabei müssen erfindungsgemäße extrudierte Depotformen nicht zwingend vor der Aufnahme in den Applikator sterilisiert werden, sondern können auch innerhalb des Applikators einem Sterilisierungsvorgang unterzogen werden. Ferner ist ein solcher Applikator in der Lage, extrudierte Depotformen unterschiedlicher Länge aufzunehmen. Dabei können selbstverständlich auch Depotformen, welche keine zylindrische Form aufweisen, sondern beispielsweise quaderförmig oder rund oder dergleichen sind, appliziert werden.

Zweckmäßig weist ein solcher Applikator eine Hohlnadel zur Aufnahme extrudierter Depotformen mit den oben erläuterten Ausmaßen und eine Schutzkappe auf, welche vor der Applikation zu entfernen ist und nach der Anwendung wieder fixiert werden kann. Dadurch wird die extrudierte Depotform vorteilhaft vor äußeren Einflüssen, welche die bevorzugte Anwendung in irgendeiner Weise negativ beeinflussen können, geschützt.

Weitere Merkmale der Erfindung ergeben sich aus der nachfolgenden Beschreibung von Ausführungsbeispielen in Verbindung mit den Ansprüchen sowie der Figuren. Es sei darauf hingewiesen, dass die Erfindung nicht auf die Ausführungsformen der beschriebenen Ausführungsbeispiele beschränkt, sondern durch den Umfang der beiliegenden Patentansprüche bestimmt ist. Insbesondere können die einzelnen Merkmale bei erfindungsgemäßen Ausführungsformen in anderer Kombination als bei den untenstehend angeführten Beispielen verwirklicht sein. Bei der nachfolgenden Erläuterung einiger Ausführungsbeispiele der Erfindung wird auf die beiliegenden Figuren Bezug genommen. Es zeigt:

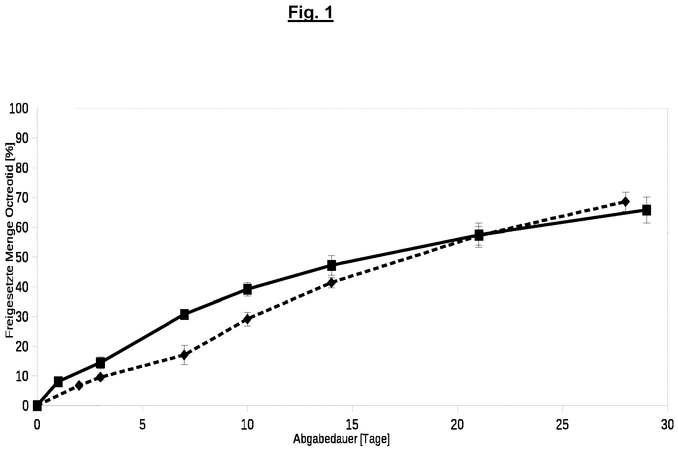

Figur 1: Freisetzungsprofile einer erfindungsgemäßen Octreotid-haltigen Depotform (gemäß Beispiel 1, siehe unten) im Vergleich mit einer nicht erfindungsgemäßen Octreotid-haltigen Depotform. Die Depotformen weisen einen Durchmesser von etwa 2 mm auf. Durchgezogene Linie (erfindungsgemäßes Beispiel): 33 Gew.-% Octreotid, 32 Gew.-% Polymilchsäure-co-glykolsäure Polymer, 32 Gew.-% Polymilchsäure, 3 Gew.-% Triglycerid. Gestrichelte Linie (Vergleichsbeispiel): 33 Gew.-% Octreotid; 33,5 Gew.-% Polymilchsäure-co-glykolsäure Polymer, 33.5 Gew.-% Polymilchsäure.

### Beispiele

### Erfindungsgemäßes Beispiel 1:

Für die Herstellung erfindungsgemäßer Depotformen wurde eine Pulvermischung eingewogen, welche zu 32 Gew.-% aus dem Polymilchsäure-co-glykolsäure Polymer (Resomer^{®} RG752H, Evonik Industries Deutschland), zu 32 Gew.-% aus Polymilchsäure (Resomer^{®} R202H, Evonik Industries Deutschland), zu 3 Gew.-% aus einem Triglycerid (Dynasan 118, IOI Oleo GmbH, Hamburg, Deutschland) und zu 33 Gew.-% aus Octreotid-Lyophilisat (Bachem, Bubendorf, Schweiz), bestand. Diese Pulvermischung wurde mittels Kryovermahlung (Freezer/Mill, C3 Prozess- und Analysentechnik GmbH, Haar bei München, Deutschland) homogen vermischt. Die nachfolgende Extrusion wurde über Gegenläuferschnecken-Schmelzextrusion (Mini CTW, Thermo Fisher Scientific GmbH, Karlsruhe, Deutschland) bei 85 °C bis 92 °C und einer Schneckendrehzahl von 8 Upm (Umdrehungen pro Minute) durchgeführt. Der Durchmesser für das Extrudat wurde mit einer Düse auf 2,0 mm eingestellt. Der Extrudatstrang wurde zu Extrudaten geeigneter Länge (im vorliegenden Beispiel einer Länge von 2 cm) geschnitten. Alternativ kann das Extrudat über Sphäronisation zu Mikropartikeln geformt werden.

### Erfindungsgemäßes Beispiel 2:

Die Herstellung erfolgte gemäß Beispiel 1, die Zusammensetzung der erfindungsgemäßen Depotformen wurde jedoch mit Calciumchlorid (Sigma Aldrich, Wien, Österreich) ergänzt. Die Pulvermischung bestand aus 30 Gew.-% Polymilchsäure-co-glykolsäure Polymer (Resomer^{®} RG752H, Evonik Industries Deutschland), 30 Gew.-% Polymilchsäure (Resomer^{®} R202H, Evonik Industries Deutschland), 3,5 Gew.-% Triglycerid (Dynasan 118, IOI Oleo GmbH, Hamburg, Deutschland), 3.5 Gew.-% Calciumchlorid und 33 Gew.-% aus Octreotid-Lyophilisat.

### Erfindungsgemäßes Beispiel 3:

Die Herstellung erfolgte gemäß Beispiel 1, die Zusammensetzung der erfindungsgemäßen Depotformen wurde jedoch mit Trehalose (Sigma Aldrich, Wien, Österreich) ergänzt. Die Pulvermischung bestand aus 30 Gew.-% Polymilchsäure-co-glykolsäure Polymer (Resomer^{®} RG752H, Evonik Industries Deutschland), 30 Gew.-% Polymilchsäure (Resomer^{®} R202H, Evonik Industries Deutschland), 3,5 Gew.-% Triglycerid (Dynasan 118, IOI Oleo GmbH, Hamburg, Deutschland), 3,5 Gew.-% Trehalose und 33 Gew.-% aus Octreotid-Lyophilisat.

### Erfindungsgemäßes Beispiel 4:

Für die Herstellung erfindungsgemäßer Depotformen wurde eine Pulvermischung aus 57 Gew.-% Polymilchsäure-co-glykolsäure Polymer (Resomer^{®} RG752H, Evonik Industries Deutschland), 10 Gew.-% Glycerol dibehenate (Compritol 888 ATO, Gattefosse) und 33 Gew.-% Octreotid-Lyophilisat hergestellt. Die Bestandteile dieser Pulvermischung wurden mittels Kryovermahlung (Freezer/Mill, C3 Prozess- und Analysentechnik GmbH, Haar bei München, Deutschland) homogen vermischt. Die folgende Extrusion wurde über Gleichläuferschnecken-Schmelzextrusion (Mini CTW, Thermo Fisher Scientific GmbH, Karlsruhe, Deutschland) bei 83 °C bis 90 °C und einer Schneckendrehzahl von 8 Upm durchgeführt.

### Erfindungsgemäßes Beispiel 5:

Für die Herstellung erfindungsgemäßer Depotformen wurde eine Pulvermischung aus 28,5 Gew.-% Polymilchsäure-co-glykolsäure (Resomer^{®} RG502H, Evonik Industries Deutschland), 28,5 Gew.-% Polymilchsäure (Resomer^{®} R203H, Evonik Industries Deutschland), 3 Gew.-% Triglycerid (Dynasan 118, IOI Oleo GmbH, Hamburg, Deutschland) und 40 Gew.-% Lanreotid mittles Kroyvermahlung zu einer homogenen Mischung verarbeitet. Für die Untersuchung der Wirkstofffreisetzung wurden beispielhafte erfindungsgemäße Depotformen in der jeweils geeigneten Form und Größe (z. B. geschnitten zu Zylindern von 1,5 bis 2 cm Länge und einem Durchmesser von 1,8 mm) zunächst einzeln gewogen.

### Erfindungsgemäßes Beispiel 6:

Für die Herstellung erfindungsgemäßer Depotformen wurde eine Pulvermischung eingewogen, welche zu 38 Gew.-% aus dem Polymilchsäure-co-glykolsäure Polymer (Resomer^{®} RG752H, Evonik Industries Deutschland), zu 26 Gew.-% aus Polymilchsäure (Resomer^{®} R202H, Evonik Industries Deutschland), zu 3 Gew.-% aus einem Triglycerid (Dynasan 118, IOI Oleo GmbH, Hamburg, Deutschland) und zu 33 Gew.-% aus Octreotid-Lyophilisat (Bachem, Bubendorf, Schweiz) bestand. Diese Pulvermischung wurde mittels Kryovermahlung (Freezer/Mill, C3 Prozess- und Analysentechnik GmbH, Haar bei München, Deutschland) homogen vermischt. Die folgende Ko-Extrusion wurde mit Hilfe einer Zweistoffdüse durchgeführt, wobei neben der eigentlichen Pulvermischung mit Hilfe eines baugleichen, zweiten Extruders (Mini CTW, Thermo Fisher Scientific GmbH, Karlsruhe, Deutschland) ein homogener Überzug aus 100 Gew.-% Polymilchsäure-co-glykolsäure Polymer (Resomer^{®} RG752H, Evonik Industries Deutschland) extrudiert wurde. Der Durchmesser für das Kern-Extrudat wurde auf 2,0 mm eingestellt und die Auftragsdicke des Überzugs wurde mit Hilfe der Zweistoffdüse auf 0,1 mm eingestellt. Der Extrudatstrang wurde im Anschluss zu Extrudaten geeigneter Länge geschnitten.

### Vergleichsbeispiel:

Für die Herstellung einer Depotform, welche als Vergleichsbeispiel dient, wurde eine Pulvermischung hergestellt, welche zu 33,5 Gew.-% aus dem Polymilchsäure-co-glykolsäure Polymer (Resomer^{®} RG752H, Evonik Industries Deutschland), zu 33,5 Gew.-% aus Polymilchsäure (Resomer^{®} R202H, Evonik Industries Deutschland) und zu 33 Gew.-% aus Octreotid-Lyophilisat (Bachem, Bubendorf, Schweiz) bestand. Diese Pulvermischung wurde mittels Kryovermahlung (Freezer/Mill, C3 Prozess- und Analysentechnik GmbH, Haar bei München, Deutschland) homogen vermischt.

Die nachfolgende Extrusion wurde über Gegenläuferschnecken-Schmelzextrusion (Mini CTW, Thermo Fisher Scientific GmbH, Karlsruhe, Deutschland) bei 85 °C bis 92 °C und einer Schneckendrehzahl von 5 - 15 Upm (Umdrehungen pro Minute) durchgeführt. Der Durchmesser für das Extrudat wurde mit einer Düse auf 2.0 mm eingestellt. Der Extrudatstrang wurde zu Extrudaten geeigneter Länge (im vorliegenden Beispiel einer Länge von 2,0 cm) geschnitten.

### Bestimmung der in vitro Freisetzung des Wirkstoffs aus den Depotformen (Beispiele 1 bis 6 sowie Vergleichsbeispiel):

Um die *in vitro* Freisetzung des Wirkstoffes aus den Depotformen zu untersuchen, wurden die Depotformen (gemäß Beispiel 1 - 6) in Freisetzungszellen gegeben und mit 50 ml Freisetzungsmedium (Dinatriumhydrogenphosphat, pH 7,4) versetzt. Die Depotformen wurden im Anschluss in einen Inkubationsschüttler (IKA^{®}-Werke GmbH & Co. KG, Deutschland) bei 37 °C über die gewünschte Applikationszeit gegeben.

Zu den jeweiligen Probenahmezeitpunkten wurden ca. 1 ml Probelösung mit einer Einmalpipette entnommen und direkt in ein HPLC-Gefäß abgefüllt. Nach jedem Probenahmezeitpunkt erfolgt ein kompletter Ersatz des Freisetzungsmediums.

### Erfindungsgemäßes Beispiel 7:

Für die Herstellung erfindungsgemäßer Depotformen wurde eine Pulvermischung eingewogen, welche zu 42 Gew.-% aus dem Polymilchsäure-co-glykolsäure Polymer (Resomer^{®} RG752H, Evonik Industries Deutschland), zu 42 Gew.-% aus Polymilchsäure (Resomer^{®} R202H, Evonik Industries Deutschland), zu 5,5 Gew.-% aus einem Triglycerid (Dynasan 118, IOI Oleo GmbH, Hamburg, Deutschland), zu 10 Gew.-% aus mRNA (CleanCap^{®} EGFP mRNA, TriLink Biotechnologies, Inc. USA) bestand und zu 0.5 Gew.-% aus Lipase (Triacylglycerol Lipase aus Pseudomonas sp., Merck KGaA Darmstadt). Die nachfolgende Extrusion wurde über Gleichläuferschnecken-Schmelzextrusion (Mini Extruder ZE-5, Three-Tec GmbH, Birnen Schweiz) bei 85 °C bis 90 °C und einer Schneckendrehzahl von 10 Upm (Umdrehungen pro Minute) durchgeführt. Der Durchmesser für das Extrudat wurde mit einer Düse auf 0,5 mm eingestellt. Der Extrudatstrang wurde zu Extrudaten geeigneter Länge (im vorliegenden Beispiel einer Länge von 0,5 cm) geschnitten.

Die Expressionskontrolle (Nachweis der GFP Aktivität) erfolgte mit Hilfe des GFP Assay System (Arbor Assays LLC, USA). Dabei wird nach Protokoll gearbeitet: Die erfindungsgemäße Depotform wird zu einer humanen Zellkultur gegeben. Die Freisetzung aus der Depotform und die Aktivität des codierten Proteins wurden über den Fluoreszenz Assay nachgewiesen. Bei Anregung mit Licht der Wellenlänge 395 nm emittierten die Zellen, welche die mRNA aufgenommen haben und das GFP Gen exprimieren, messbares Licht mit der Wellenlänge 509 nm. In Zellkulturen, welche eine Negativkontrolle (ohne mRNA) erhalten hatten, konnte keine wesentliche Fluoreszenz gemessen werden.

### Erfindungsgemäßes Beispiel 8:

Die Herstellung erfolgte gemäß Beispiel 1, die Zusammensetzung der erfindungsgemäßen Depotformen wurde jedoch mit Glycerol dibehenate ergänzt. Die Pulvermischung bestand aus 40 Gew.-% Polymilchsäure-co-glykolsäure Polymer (Resomer^{®} RG752H, Evonik Industries Deutschland), 40 Gew.-% Polymilchsäure (Resomer^{®} R202H, Evonik Industries Deutschland), 5 Gew.-% Triglycerid (Dynasan 118, IOI Oleo GmbH, Hamburg, Deutschland), 4,5 Gew.-% Glycerol dibehenate (Compritol 888 ATO, Gattefosse), zu 10 Gew.-% aus mRNA (CleanCap^{®} EGFP mRNA, TriLink Biotechnologies, Inc. USA) und zu 0.5 Gew.-% aus Lipase (Triacylglycerol Lipase aus Pseudomonas sp., Merck KGaA Darmstadt). Die Expressionskontrolle erfolgte analog zu Beispiel 7.

### Erfindungsgemäßes Beispiel 9:

Für die Herstellung erfindungsgemäßer Depotformen wurde eine Pulvermischung eingewogen, welche zu 74,5 Gew.-% aus dem Polymilchsäure-co-glykolsäure Polymer (Resomer^{®} RG752H, Evonik Industries Deutschland), zu 15 Gew.-% aus einem Triglycerid (Dynasan 118, IOI Oleo GmbH, Hamburg, Deutschland), zu 10 Gew.-% aus mRNA (CleanCap^{®} FLuc mRNA, TriLink Biotechnologies, Inc. USA) bestand und zu 0.5 Gew.-% aus Lipase (Triacylglycerol Lipase aus Pseudomonas sp., Merck KGaA Darmstadt). Diese Pulvermischung wurde mittels Kryovermahlung (Freezer/Mill, C3 Prozess- und Analysentechnik GmbH, Haar bei München, Deutschland) homogen vermischt. Die nachfolgende Extrusion wurde über Gleichläuferschnecken-Schmelzextrusion (Mini Extruder ZE-5, Three-Tec GmbH, Birnen Schweiz) bei 83 °C bis 92 °C und einer Schneckendrehzahl von 10 Upm durchgeführt.

Die Expressionskontrolle (Nachweis der Luciferase Aktivität) erfolgte mit Hilfe des Luciferase Assay System E1500 (Promega Corporation, USA). Dabei wurde nach Protokoll gearbeitet: Die erfindungsgemäße Depotform wurde zu einer humanen Zellkultur gegeben. Die Freisetzung aus der Depotform und die Aktivität des codierten Proteins wurden über den Luciferase Assay nachgewiesen. Bei Zugabe des Substrates (Luciferin) emittierten die Zellen, welche die mRNA aufgenommen haben und das Luciferase Enzym exprimieren, messbares Licht. In Zellkulturen, welche eine Negativkontrolle (ohne mRNA) erhalten hatten, konnte keine wesentliche Lumineszenz gemessen werden.

## Patentansprüche

1. Eine extrudierte Depotform, insbesondere zur anhaltenden Wirkstofffreisetzung, umfassend
(a) zumindest einen Wirkstoff,
(b) zumindest eine Verbindung aus der Klasse der bioabbaubaren organischen Polymeren auf Basis von Milchsäure und/oder Glykolsäure, und
(c) zumindest eine zweite Verbindung aus der Klasse der Lipide, und
(d) wobei b) und c) vorzugsweise mindestens 50 Gew.-% des Trockengewichts der Depotform umfassen,
wobei das Verhältnis der zumindest einen Verbindung aus der Klasse der bioabbaubaren organischen Polymere und der zweiten Verbindung aus der Klasse der Lipide bei höchstens etwa 25:1 und/oder bei mindestens etwa 5:1 liegt,
wobei der Schmelzpunkt der zumindest einen Verbindung aus der Klasse der Lipide bei mindestens etwa 60 °C und/oder bei höchstens etwa 80 °C liegt,
und
wobei die Depotform eine Länge von mindestens 0,1 cm und/oder von höchstens 5 cm aufweist.

2. Extrudierte Depotform gemäß Anspruch 1, wobei das bioabbaubare Polymer auf Basis von Milchsäure und/oder Glykolsäure ausgewählt ist aus Poly(L-lactid), Poly(D, L-lactid), Poly(glycolid), Poly(L-lactid-co-D,L-lactid), Poly(L-lactid-co-glycolid), Poly(D,L-lactid-co-glycolid), Poly(meso-lactid), Poly(D,L-lactid-co-trimethylencarbonat), Poly(L-lactid-co-meso-lactid), Poly(L-lactid-co-epsilon-caprolacton), Poly(D, L-lactid-co-meso-lactid), Poly(D, L-lactid-co- epsilon-caprolacton), Poly(meso-lactid-co-glycolid), Poly(meso-lactid-co-trimethylencarbonat), Poly(meso-lactid-co-epsilon-caprolacton), Poly(glycolid-co-trimethylencarbonat), Poly(glycolid-co-epsilon-caprolacton) und/oder Poly(glycolid-co-caprolacton).

3. Extrudierte Depotform gemäß einem der vorhergehenden Ansprüche, wobei die zumindest eine Verbindung aus der Klasse der Lipide, vorzugsweise der Hartfette, besonders bevorzugt der Mono-, Di- und/oder Triglyceride und Salzen und Derivaten davon, ausgewählt ist.

4. Extrudierte Depotform gemäß einem der vorhergehenden Ansprüche, wobei die zumindest eine Verbindung aus der Klasse der Lipide aus Veresterung von Glycerin mit gesättigten und/oder ungesättigten Fettsäuren mit einer Länge von 5 bis 26 Kohlenstoffatomen ausgewählt ist.

5. Extrudierte Depotform gemäß einem der vorhergehenden Ansprüche, wobei die extrudierte Depotform ferner einen oder mehrere Hilfsstoff/e umfasst, welche/r ausgewählt ist/sind aus der Klasse der Enzyme, bevorzugt aus Lipasen; Polyethylenoxide, wie beispielsweise Polyethylenglykol; Salze mit divalenten Metallionen, bevorzugt Calciumchlorid; Disaccharide, bevorzugt Trehalose; Oligosaccharide, bevorzugt Cyclodextrine; Polysaccharide, bevorzugt Cellulose.

6. Extrudierte Depotform gemäß einem der vorhergehenden Ansprüche, wobei der zumindest eine Wirkstoff ausgewählt ist aus der Klasse der Peptidhormone, bevorzugt aus Octreotid, Pasireotid, Lanreotid und/oder Valpreotid, insbesondere aus Octreotid, aus der Klasse der Wachstumshormone, bevorzugt aus Somatotropin und/oder Erythropoetin, aus der Klasse der VEGF-Inhibitoren, bevorzugt aus Bevacizumab und/oder Ranibizumab, aus der Klasse der Antidiabetika, bevorzugt aus Exenatid, aus der Klasse der Gonadotropin-Releasing-Hormon-Analoga (GnRH-Analoga), bevorzugt aus Goserelin und/oder Leuprorelin, aus der Klasse der Antirheumatika, bevorzugt aus Infliximab und/oder Etanercept, aus der Klasse der Brustkrebstherapeutika, bevorzugt aus Trastuzumab, aus der Klasse der MS (Multiple Sklerose)-Therapeutika, bevorzugt aus Natalizumab, aus der Klasse der Programmierten Zelltod-Rezeptor-1 (PD-1) Antagonisten, bevorzugt aus Pembrolizumab, aus der Klasse der Proteinarzneistoffe gegen die paroxysmale nächtliche Hämoglobinurie, bevorzugt aus Eculizumab, aus der Klasse der Neuroleptika, bevorzugt aus Risperidon.

7. Extrudierte Depotform gemäß einem der vorhergehenden Ansprüche, wobei der zumindest eine Wirkstoff ausgewählt ist aus Nucleinsäuren, bevorzugt aus der Klasse von DNA, Plasmid-DNA, kationischen DNA-Komplexen und/oder RNA, siRNA, mRNA, besonders bevorzugt aus RNA, siRNA und/oder mRNA, insbesondere aus selbstreplizierender RNA und/oder mRNA.

8. Extrudierte Depotform gemäß einem der vorhergehenden Ansprüche, wobei die extrudierte Depotform einen homogenen Kernüberzug aufweist, der eine homogene Zusammensetzung der Komponenten gemäß Anspruch 1 a), b) und/oder c) aufweist.

9. Extrudierte Depotform gemäß einem der vorhergehenden Ansprüche, wobei die extrudierte Depotform ein Verhältnis von Durchmesser zu Länge von mindestens etwa 1:30 und/oder höchstens etwa 10:1, bevorzugt ein Verhältnis von Durchmesser zu Länge von mindestens etwa 1:15 und/oder höchstens etwa 5:1, aufweist.

10. Verfahren zur Herstellung einer extrudierten Depotform gemäß einem der vorhergehenden Ansprüche umfassend die Schritte
(i) Bereitstellen einer vorzugsweise homogenen Mischung gemäß einem der Ansprüche 1 bis 9,
(ii) Extrusion, insbesondere Schmelzextrusion, des Kerns bei einer Temperatur oberhalb der Schmelztemperatur des zumindest einen Lipids,
(iii) optional Aufbringen einer Überzugsmischung bzw. -zusammensetzung auf das vorstehend erhaltene Extrudat vorzugsweise gleichzeitig mit Schritt (ii),
(iv) Schneiden des Extrudats in Stücke geeigneter Größe,
(v) optional Runden der Stücke, insbesondere durch Sphäronisation,
(vi) optional Durchführen eines Sterilisierungsvorgangs und/oder Verpacken der Depotform.

11. Extrudierte Depotform gemäß einem der Ansprüche 1 bis 9, wobei die extrudierte Depotform nach einem Verfahren gemäß Anspruch 10 erhältlich ist.

12. Extrudierte Depotform gemäß einem der Ansprüche 1 bis 9, wobei die in vitro Freisetzung des bevorzugten Peptidhormons, insbesondere von Octreotid, nach 7 Tagen bei mindestens etwa 20 Gew.-% und/oder nach 28 Tagen bei mindestens etwa 60 Gew.-% liegt.

13. Extrudierte Depotform gemäß einem der vorhergehenden Ansprüche 1 bis 9 und 11 bis 12 zur medizinischen, tiermedizinischen oder kosmetischen Anwendung.

14. Extrudierte Depotform gemäß einem der vorhergehenden Ansprüche 1 bis 9 und 11 bis 13 zur Anwendung bei der Behandlung der Akromegalie; von Symptomen, die mit (funktionell aktiven) gastroenteropankreatischen endokrinen Tumoren assoziiert sind, wie beispielsweise Karzinoide mit Merkmalen des Karzinoidsyndroms; fortgeschrittenen neuroendokrinen Tumoren; Thyreoidea-stimulierendes Hormon (TSH)-sezernierende Hypophysenadenome; zur Vakzinierung und/oder Krebstherapie; zur Therapie bei nicht-funktionaler Expression von Proteinen und/oder Enzymen; zur Behandlung der Makuladegeneration und/oder des Glaukoms, von Diabetes bedingten Augenerkrankungen sowie onkologisch bedingten Augenerkrankungen.

## Claims

1. An extruded depot form, in particular for sustained active substance release, comprising
(a) at least one active substance,
(b) at least one compound from the class of biodegradable organic polymers based on lactic acid and/or glycolic acid, and
(c) at least one second compound from the class of lipids, and
(d) wherein b) and c) preferably comprise at least 50 % by weight of the dry weight of the depot form,
wherein the ratio of the at least one compound from the class of biodegradable organic polymers and the second compound from the class of lipids is at most about 25:1 and/or at least about 5:1,
wherein the melting point of the at least one compound from the class of lipids is at least approximately 60 °C and/or at most approximately 80 °C
and
wherein the extruded depot form has a length of at least 0.1 cm and/or at most 5 cm.

2. Extruded depot form as claimed in claim 1, wherein the biodegradable polymer on the basis of lactic acid and/or glycolic acid is selected from poly(L-lactide), poly(D,L-lactide), poly(glycolide), poly(L-lactide-co-D,L-lactide), poly(L-lactide-co-glycolide), poly(D,L-lactide-co-glycolide), poly(meso-lactide), poly(D,L-lactide-co-trimethylene carbonate), poly(L-lactide-co-meso-lactide), poly(L-lactide-co-epsilon-caprolactone), poly(D,L-lactide-co-meso-lactide), poly(D,L-lactide-co-epsilon-caprolactone), poly(meso-lactide-co-glycolide), poly(meso-lactide-co-trimethylene carbonate), poly(meso-lactide-co-epsilon-caprolactone), poly(glycolide-co-trimethylene carbonate), poly(glycolide-co-epsilon-caprolactone) and/or poly(glycolide-co-caprolactone).

3. Extruded depot form as claimed in one of the preceding claims, wherein the at least one compound is selected from the class of lipids, preferably from hard fats, particularly preferably from mono-, di- and/or triglycerides and salts and derivatives thereof.

4. Extruded depot form as claimed in one of the preceding claims, wherein the at least one compound from the class of lipids is selected from esters of glycerine with saturated and/or unsaturated fatty acids with a length of 5 to 26 carbon atoms.

5. Extruded depot form as claimed in one of the preceding claims, wherein the extruded depot form furthermore comprises one or more excipient/s which is/are selected from the class of enzymes, preferably from lipases; polyethylene oxides such as for example polyethylene glycol; salts with divalent metal ions, preferably calcium chloride; disaccharides, preferably trehalose; oligosaccharides, preferably cyclodextrins; polysaccharides, preferably cellulose.

6. Extruded depot form as claimed in one of the preceding claims, wherein the at least one active substance is selected from the class of peptide hormones, preferably from octreotide, pasireotide, lanreotide and/or valpreotide, in particular from octreotide, from the class of growth hormones, preferably from somatotropin and/or erythropoetin, from the class of VEGF inhibitors, preferably from bevacizumab and/or ranibizumab, from the class of antidiabetics, preferably from exenatide, from the class of gonadotropin-releasing hormone analogues (GnRH analogues), preferably from goserelin and/or leuprorelin, from the class of antirheumatics, preferably from infliximab and/or etanercept, from the class of breast cancer therapeutics, preferably from trastuzumab, from the class of MS (multiple sclerosis) therapeutics, preferably from natalizumab, from the class of programmed cell death receptor-1 (PD-1) antagonists, preferably from pembrolizumab, from the class of protein medicinal products against paroxysmal nocturnal haemoglobulinuria, preferably from eculizumab, from the class of neuroleptics, preferably from risperidone.

7. Extruded depot form as claimed in one of the preceding claims, wherein the at least one active substance is selected from nucleic acids, preferably from the class of DNA, plasmid DNA, cationic DNA complexes and/or RNA, siRNA, mRNA, particularly preferably from RNA, siRNA and/or mRNA, in particular from self-replicating RNA and/or mRNA.

8. Extruded depot form as claimed in one of the preceding claims, wherein the extruded depot form comprises a homogeneous core coating which comprises a homogeneous composition of the components as claimed in claim 1 a), b) and/or c).

9. Extruded depot form as claimed in one of the preceding claims, wherein the extruded depot form has a ratio of diameter to length of at least approximately 1:30 and/or at most approximately 10:1, preferably a ratio of diameter to length of at least approximately 1:15 and/or at most approximately 5:1.

10. A method for the production of an extruded depot form as claimed in one of the preceding claims, comprising the following steps:
(i) providing a preferably homogeneous mixture as claimed in one of the claims 1 to 9,
(ii) extruding the core, in particular by melt extrusion, at a temperature above the melting temperature of the at least one lipid,
(iii)optionally, applying a coating mixture or composition to the above-obtained extrudate, preferably simultaneously with step (ii),
(iv)cutting the extrudate into pieces of suitable size,
(v) optionally, rounding the pieces, in particular by spheronisation,
(vi)optionally, carrying out a sterilisation procedure and/or packaging of the depot form.

11. The extruded depot form as claimed in one of claims 1 to 9, wherein the extruded depot form is obtainable in accordance with a method as claimed in claim 10.

12. The extruded depot form as claimed in one of claims 1 to 9, wherein the in vitro release of the preferred peptide hormone, in particular of octreotide, is at least approximately 20 % by weight after 7 days and/or is at least approximately 60 % by weight after 28 days.

13. The extruded depot form as claimed in one of the preceding claims 1 to 9 and 11 to 12, for medical, veterinary or cosmetic application.

14. The extruded depot form as claimed in one of the preceding claims 1 to 9 and 11 to 13, for use in the treatment of acromegaly; of symptoms which are associated with (functionally active) gastroenteropancreatic endocrinal tumours such as, for example, carcinoids with features of carcinoid syndrome; advanced neuroendocrinal tumours; thyroidea-stimulating hormone (TSH)-secreting pituitary adenomas; for vaccination and/or cancer therapy; for therapy in the non-functional expression of proteins and/or enzymes; for the treatment of macular degeneration and/or glaucoma, for diabetes-related eye diseases as well as oncological-related eye diseases.

## Revendications

1. Forme extrudée à réservoir, destinée notamment à une libération prolongée de principes actifs, comprenant
(a) au moins un principe actif,
(b) au moins un composé issu de la catégorie des polymères organiques biodégradables à base d'acide lactique et/ou d'acide glycolique, et
(c) au moins un second composé issu de la catégorie des lipides, et
(d) b) et c) constituant préférentiellement au moins 50 % en poids du poids sec de la forme à réservoir,
le rapport entre l'au moins un composé issu de la catégorie des polymères organiques biodégradables et le second composé issu de la catégorie des lipides étant inférieur ou égal à 25:1 et supérieur ou égal à 5:1,
le point de fusion de l'au moins un composé issu de la catégorie des lipides étant supérieur ou égal à environ 60 °C et/ou inférieur ou égal à environ 80 °C,
et
la forme à réservoir ayant une longueur supérieure ou égale à 0,1 cm et/ou inférieure ou égale à 5 cm.

2. Forme à réservoir extrudée selon la revendication 1, le polymère biodégradable à base d'acide lactique et/ou d'acide glycolique étant choisi parmi le poly(L-lactide), le poly(D,L-lactide), le poly(glycolide), le poly(L-lactide-co-D,L-lactide), le poly(L-lactide-co-glycolide), le poly(D,L-lactide-coglycolide), le poly(meso-lactide), le poly(D,L-lactide-cotriméthylencarbonate), le poly(L-lactide-co-meso-lactide), la poly(L-lactideco-epsilon-caprolactone), le poly(D,L-lactide-co-meso-lactide), la poly(D,L-lactide-co-epsilon-caprolactone), le poly(meso-lactide-co-glycolide), le poly(meso-lactide-co-triméthylencarbonate), la poly(meso-lactide-coepsilon-caprolactone), le poly(glycolide-co-triméthylencarbonate), la poly(glycolide-co-epsilon-caprolactone) et/ou la poly(glycolide-cocaprolactone).

3. Forme à réservoir extrudée selon l'une des revendications précédentes, l'au moins un composé issu de la catégorie des lipides, préférentiellement des lipides durs, étant choisi avec une préférence particulière parmi les mono-, di- et/ou triglycérides et leurs sels et dérivés.

4. Forme à réservoir extrudée selon l'une des revendications précédentes, l'au moins un composé issu de la catégorie des lipides étant choisi parmi esters que le glycérol forme avec des acides gras saturés et/ou insaturés ayant une longueur comprise entre 5 et 26 atomes de carbone.

5. Forme à réservoir extrudée selon l'une des revendications précédentes, la forme à réservoir extrudée comprenant en outre un ou plusieurs agent(s) auxiliaire(s) le(s)quel(s) est/sont choisi(s) dans la catégorie des enzymes, s'agissant préférentiellement de lipases; des polyoxyéthylènes tels que, par exemple, le polyéthylène-glycol; des sels formés avec des ions métalliques divalents, s'agissant préférentiellement de chlorure de calcium; des disaccharides, s'agissant préférentiellement de tréhalose; des oligosaccharides, s'agissant préférentiellement de cyclodextrines; des polysaccharides, s'agissant préférentiellement de cellulose.

6. Forme à réservoir extrudée selon l'une des revendications précédentes, l'au moins un principe actif étant choisi dans la catégorie des hormones peptidiques, de préférence parmi l'octréotide, le pasiréotide, le lanréotide et/ou le valpréotide, s'agissant notamment d'octréotide, dans la catégorie des hormones de croissance, de préférence parmi la somatotropine et/ou l'érythropoétine, dans la catégorie des inhibiteurs de VEGF, de préférence parmi le bévacizumab et/ou le ranibizumab, dans la catégorie des analogues de l'hormone de libération des gonadotropines (analogues de GnRH), de préférence parmi la goséréline et/ou la leuproréline, dans la catégorie de antirhumatismaux, de préférence parmi l'infliximab et/ou l'étanercept, dans la catégorie des thérapeutiques du cancer du sein, s'agissant préférentiellement de natalizumab, dans la catégorie des antagonistes du récepteur de la mort cellulaire programmée (PD-1), s'agissant préférentiellement de pembrolizumab, dans la catégorie des produits pharmaceutiques protéiniques contre l'hémoglobinurie paroxystique nocturne, s'agissant préférentiellement d'éculizumab, dans la catégorie des neuroleptiques, s'agissant préférentiellement de rispéridone.

7. Forme à réservoir extrudée selon l'une des revendications précédentes, l'au moins un principe actif étant choisi parmi les acides nucléiques, de préférence dans la catégorie des ADN, des ADN plasmidiques, des complexes cationiques d'ADN et/ou des ARN, des pARNi, des ARNm, avec une préférence particulière parmi les ARN, les pARNi et/ou les ARNm, notamment parmi les ARN auto-répliquants et/ou les ARNm.

8. Forme à réservoir extrudée selon l'une des revendications précédentes, la forme à réservoir extrudée présentant un enrobage de noyau homogène ayant une composition homogène des constituants selon la revendication 1 a), b) et/ou c).

9. Forme à réservoir extrudée selon l'une des revendications précédentes, la forme à réservoir extrudée ayant un rapport entre le diamètre et la longueur qui est supérieur ou égal à environ 1:30 et/ou inférieur ou égal à environ 10:1, de préférence un rapport entre le diamètre et la longueur qui est supérieur ou égal à environ 1:15 et/ou inférieur ou égal à environ 5:1.

10. Procédé de fabrication d'une forme à réservoir extrudée selon l'une des revendications précédentes, comprenant les étapes consistant à
(i) fournir un mélange préférentiellement homogène selon l'une des revendications 1 à 9,
(ii) extruder, notamment par extrusion à l'état fondu, le noyau à une température supérieure à la température de fusion de l'au moins un lipide,
(iii) optionnellement, appliquer un mélange ou une composition d'enrobage - sur l'extrudat précédemment obtenu, de préférence simultanément avec l'étape (ii),
(iv) découper ledit extrudat en morceaux de taille appropriée,
(v) optionnellement, arrondir lesdits morceaux, notamment par sphéronisation,
(vi) optionnellement, réaliser un processus de stérilisation et/ou emballer la forme à réservoir.

11. Forme à réservoir extrudée selon l'une des revendications 1 à 9, la forme à réservoir extrudée pouvant être obtenue suivant un procédé selon la revendication 10.

12. Forme à réservoir extrudée selon l'une des revendications 1 à 9, la libération in vitro de l'hormone peptidique préférée, s'agissant notamment d'octréotide, étant au terme de 7 jours à au moins environ 20 % en poids et/ou au terme de 28 jours à au moins environ 60 % en poids.

13. Forme à réservoir extrudée selon l'une des revendications précédentes 1 à 9 et 11 à 12, destinée à une utilisation médicale, vétérinaire ou cosmétique.

14. Forme à réservoir extrudée selon l'une des revendications précédentes 1 à 9 et 11 à 13, destinée à être utilisée dans le traitement de l'acromégalie; de symptômes associés à des tumeurs (fonctionnellement actif) endocriniennes gastro-entéro-pancréatique, telles que les tumeurs carcinoïdes ayant les caractéristiques du syndrome carcinoïde; de tumeurs endocriniennes au stade avancé; de l'adénome de l'hypophyse secrétant l'hormone thyréostimuline (TSH); dans la vaccination et/ou la thérapie visant le cancer; dans la thérapie d'une expression non-fonctionnelle de protéines et/ou d'enzymes; dans le traitement de la dégénérescence maculaire et/ou du glaucome, de maladies oculaires associées au diabète ainsi que de maladies oculaires d'origine oncologique.
